# EUROPÄISCHE PATENTSCHRIFT

(11) **EP 3 496 792 B1**
(45) Veröffentlichungstag und Bekanntmachung des Hinweises auf die Patenterteilung: **09.03.2022**
(21) Anmeldenummer: 17751068.2
(22) Anmeldetag: 03.08.2017
(51) Int. Cl.: A61M 15/06, A61M 11/04, A24F 47/00, A61M 16/00

(54) **VERFAHREN ZUM BETREIBEN EINES INHALATORS**
METHOD OF OPERATING AN INHALER
MÉTHODE DE COMMANDE D'UN INHALATEUR

(30) Priorität: 09.08.2016 DE 102016114718
(43) Veröffentlichungstag der Anmeldung: 19.06.2019
(73) Patentinhaber: Hauni Maschinenbau GmbH, 21033 Hamburg (DE)
(72) Erfinder: KESSLER, Marc, 22415 Hamburg (DE); SCHMIDT, Rene, 21244 Buchholz i.d.N. (DE)
(74) Vertreter: Müller Verweyen
(86) Internationale Anmeldenummer: PCT/EP2017/069667
(87) Internationale Veröffentlichungsnummer: WO 2018/029077

(56) Entgegenhaltungen:
- WO-A1-95/01137
- WO-A1-2014/085719
- WO-A1-2014/102091
- DE-U1-202013 010 986
- US-A1- 2005 016 550
- US-A1- 2014 096 782
- US-A1- 2014 321 837
- US-A1- 2016 022 930
- US-A1- 2016 057 811
- US-A1- 2016 205 998

## Beschreibung

Die vorliegende Erfindung betrifft ein Verfahren zum Betreiben eines Inhalators umfassend ein Gehäuse mit einem Mundende, mindestens einer Lufteinlassöffnung und einem in dem Gehäuse zwischen der mindestens einen Lufteinlassöffnung und dem Mundende verlaufenden Luftkanal, eine Aufnahme für einen Flüssigkeitsspeicher, einen elektrischen Energiespeicher, eine Zugabevorrichtung zur Erzeugung von Dampf und/oder Aerosol von aus dem Flüssigkeitsspeicher entnommenem Komponentengemisch und Zugabe des Dampfs und/oder Aerosols zu einem in dem Luftkanal strömenden Luftstrom, wobei die Zugabevorrichtung einen separat ansteuerbaren Verdampfer aufweist, und mit einer Steuereinrichtung zum Steuern der Zugabevorrichtung.

Der überwiegende Teil der aktuell am Markt befindlichen elektronischen Zigarettenprodukte basiert auf dem sogenannten Dochtwendelprinzip. Ein Docht, z.B. aus Glasfaser, ist partiell mit einer Heizwendel umwickelt und steht mit einem Flüssigkeitsspeicher in Kontakt. Bei Erwärmung der Heizwendel verdampft die in dem Docht befindliche Flüssigkeit, auch als Liquid bezeichnet, im Bereich der Heizwendel. Aufgrund der Kapillarwirkung wird die verdampfte Flüssigkeit aus dem Flüssigkeitsspeicher nachgefördert. Eine solche elektronische Zigarette ist beispielhaft in der US 2016/0021930 A1 beschrieben.

Bei diesen Systemen treten eine Reihe von Problemen auf. Erstens ist die kapillare Liquidnachförderung gekoppelt an die verdampfte Liquidmenge und die physikalischen Eigenschaften der einzelnen Komponenten des Komponentengemisches. Weil die maximale Förder- und Verdampfungsleistung somit physikalisch begrenzt ist, besteht die Gefahr der Überhitzung ("hot puff"), wenn die Heizleistung die Förderleistung übersteigt.

Zweitens ist die Temperatur bei diesen Systemen physikalisch bedingt nicht eindeutig einstellbar über die Länge der Heizwendel, bzw. am Ort und über die Zeit des Verdampfens, so dass keine homogene Verdampfung im Gesamtsystem erreicht werden kann.

Drittens ist die Temperatur nicht lokal begrenzbar, woraus eine Gefahr der Überhitzung durch inhomogene Liquidbenutzung vor und während eines Zuges, und in Verbindung damit Schadstoffemission und explosionsartige Liquidfreisetzung, resultiert.

Viertens findet eine von der Ausgestaltung des Systems und von der Liquidzusammensetzung abhängige Entmischung und somit Konzentrationsänderung des Komponentengemisches statt, woraus eine unerwünschte veränderliche Wirkstofffreisetzung je Zug resultiert.

WO 95/01137 A1, US 2016/0057811A1, WO 2014/085719A1 und US 2014/0096782 A1 offenbaren jeweils einen Inhalator mit einem elektrischen Heizelement.

Die WO 2014/102091 A1 und die US 2014/0321837 A1 offenbaren ein Verfahren zum Steuern einer Aerosolproduktion in einem Aerosolerzeugungssystem.

Die US 2016/0205998 A1 offenbart einen verbrennungsfreien Aromaaspirator mit einem Zerstäuber, der dafür ausgebildet ist, die Aerosolquelle zu zerstäuben ohne zu brennen.

Die DE 20 2013 010 986 U1 offenbart eine elektronische Zigarette, bei der zum Erreichen eines gewünschten Dampfergebnisses die Heizleistung eines Heizelements kontrolliert wird.

Die US 2016/0022930 A1 offenbart ebenfalls die Steuerung eines Heizelements mittels der Heizleistung. Zur gezielten Einstellung der Dampfzusammensetzung sind zwei Kammern mit unterschiedlichen Liquiden vorgesehen, die auf unterschiedliche Temperaturen erhitzt werden können.

Die Aufgabe der Erfindung besteht darin, einen Inhalator und einen Flüssigkeitsspeicher dafür bereitzustellen, bei denen ein oder mehrere der zuvor geschilderten Nachteile vermieden werden.

Die Erfindung löst diese Aufgabe mit den Merkmalen des unabhängigen Anspruchs 1.

Demnach ist die Steuereinrichtung erfindungsgemäß mit einem Datenspeicher verbunden, in dem zum Verdampfen des Komponentengemischs in dem Verdampfer mindestens ein Satz vorgegebener Verdampfungsparameter abrufbar hinterlegt ist.

Die Erfindung hat aus theoretischen Überlegungen heraus erkannt und praktisch durch Messungen exemplarisch belegt, dass ein eindeutiger und universeller Zusammenhang zwischen Liquidzusammensetzung, Temperaturverhalten während der Verdampfung, insbesondere bedingt durch die verwendete Verdampfertechnologie, und Wirkstofffreisetzung besteht.

Ein Aspekt der Erfindung besteht in der Umsetzung dieser Erkenntnis in die Steuerung der Verdampfung des Komponentengemisches mittels des in dem Datenspeicher hinterlegten Satzes von Verdampfungsparametern. Bekannte Verdampfungstemperatur und bekannter Systemzustand stehen über die Eigenschaften des Komponentengemisches in direktem Zusammenhang miteinander. Die zu jedem Zeitpunkt der Verdampfung abgerufene Heizleistung erlaubt somit einen direkten Rückschluss auf die momentane Liquid- bzw. Dampfzusammensetzung. Diese Kenntnis kann wiederum zur Steuerung der Verdampfung des Komponentengemisches genutzt werden.

Ein weiterer Aspekt der Erfindung besteht in einer gezielten Flüssigkeitszusammensetzung zur Beeinflussung der Wirkstofffreisetzung bei gegebener bzw. eingestellter Grenztemperatur (Maximaltemperatur) des Verdampfers. Dadurch kann die Konzentrationsänderung des Komponentengemisches vollständig kontrolliert und/oder eine Entmischung der Flüssigkeitskomponenten verhindert werden. Zudem kann eine Vermeidung von Überhitzung und damit einhergehender Schadstoffemission erreicht werden.

Weitere Aspekte der Erfindung betreffen die Liquidförderung und/oder die Verdampfung. Die Liquidförderung geschieht vorteilhaft aktiv, beispielsweise mittels mikromechanischer Pumpen, oder passiv, beispielsweise durch mikrofluidische Kapillarwirkung. Die Verdampfung geschieht vorteilhaft mittels einer von der Zuförderung entkoppelten Heizeinrichtung, beispielsweise einer Heizfläche, insbesondere auf der Basis von MEMS (Mikro-Elektro-Mechanisches System), die auf die jeweils erforderliche (maximale) Verdampfungstemperatur gesteuert oder geregelt wird.

Erfindungsgemäß wird der mindestens eine Satz vorgegebener Verdampfungsparameter aus dem Flüssigphase-Gasphase-Gleichgewichts Diagramm des Komponentengemisches abgeleitet.

Vorzugsweise enthält der mindestens eine Satz vorgegebener Verdampfungsparameter Daten zu einer oder mehreren aus der Gruppe der folgenden Größen: eine einzustellende Temperatur, insbesondere die Temperatur des Verdampfers bzw. der Heizeinrichtung; eine einzustellende Dauer einzelner Verdampfungsschritte und/oder des gesamten Verdampfungsvorgangs in dem Verdampfer; einen einzustellenden Druck, insbesondere in dem Zerstäuber; eine bereitzustellende Verdampfungsenergie für den Verdampfer. Weiter vorzugsweise enthält der mindestens eine Satz vorgegebener Verdampfungsparameter Daten zu einer oder mehreren aus der Gruppe der folgenden Größen: eine minimale Temperatur und/oder eine maximale Temperatur; einen minimalen Druck und/oder einen maximalen Druck; eine minimale Dauer und/oder eine maximale Dauer einzelner Verdampfungsschritte und/oder des gesamten Verdampfungsvorgangs. Zum Einstellen einer vorgegebenen Temperatur umfasst der Verdampfer vorzugsweise eine Heizeinrichtung. Der Verdampfer kann vorteilhaft einen Temperatursensor zur Messung der Temperatur der Heizeinrichtung und/oder zur Temperatursteuerung bzw. -regelung der Heizeinrichtung aufweisen.

In einer bevorzugten Ausführungsform der Erfindung ist die vorgegebene Temperatur innerhalb eines Verdampfungsintervalls variabel, insbesondere über die Dauer eines Verdampfungsintervalls zeitweise steigend und/oder zeitweise fallend und/oder zeitweise gleichbleibend. Hierdurch kann einer unerwünschten veränderlichen Wirkstofffreisetzung, insbesondere über einen Zug, entgegengewirkt werden.

In einer weiteren bevorzugten Ausführungsform der Erfindung wird die Temperatur des Verdampfers bzw. der Heizeinrichtung mindestens über ein einem Inhalationszug entsprechendes Verdampfungsintervall oberhalb einer ersten charakteristischen Temperatur, insbesondere der Siedetemperatur einer Komponente des Komponentengemisches, gehalten. Der vorgegebene Temperaturwert liegt dabei vorteilhaft im Bereich zwischen 1 % bis 90 %, vorzugsweise 3 % bis 70 %, besonders bevorzugt 5 % bis 50 % oberhalb der ersten charakteristischen Temperatur. Dieser Aspekt ist ggf. unabhängig, d.h. in einer nur auf den Oberbegriff des Anspruchs 1 rückbezogenen Form, schützbar.

Eine Maximal-Temperatur des Verdampfers beträgt vorzugsweise höchstens 350 °C, weiter bevorzugt höchstens 300 °C und besonders bevorzugt höchstens 290 °C. Durch diese Maßnahme kann der Entwicklung von Schadstoffen aufgrund Überhitzung entgegengewirkt werden.

In einer ebenfalls bevorzugten Ausführungsform der Erfindung wird die Temperatur des Verdampfers bzw. der Heizeinrichtung mindestens über ein Verdampfungsintervall unterhalb einer zweiten charakteristischen Temperatur, insbesondere der Siedetemperatur einer Komponente des Komponentengemisches, gehalten. Dabei ist die zweite charakteristische Temperatur vorzugsweise die Siedetemperatur einer höher als eine Wirkstoffkomponente siedenden Komponente, vorteilhaft der höchstsiedenden Komponente des Komponentengemisches. Dieser Aspekt ist ggf. unabhängig, d.h. in einer nur auf den Oberbegriff des Anspruchs 1 rückbezogenen Form, schützbar. In dieser Ausführungsform kann der Anteil einer höher als eine Wirkstoffkomponente siedenden Komponente in der Art einer Stellschraube gezielt verwendet werden, um die Stärke der Wirkstoffzufuhr pro Zug zu dosieren und beispielsweise zu erhöhen gegenüber einer Stärke, die sich rein rechnerisch aus dem Massenanteil des Wirkstoffs an dem Komponentengemisch ergibt. Diese Wirkung ergibt sich daraus, dass die höher als der Wirkstoff siedende (Dummy-)Komponente nicht nennenswert verdampfen kann, so dass die Verdampferleistung auf die niedriger siedenden Komponenten einschließlich Wirkstoff konzentriert wird.

Das Verdampfungsintervall weist dabei vorzugsweise eine Dauer von 1 bis 12 Sekunden, weiter bevorzugt 2 bis 8 Sekunden auf.

In einer weiteren Ausführungsform der Erfindung ist dem Flüssigkeitsspeicher eine maschinell auslesbare Kennung des Komponentengemisches zugeordnet. Die Steuereinrichtung kann die Kennung auslesen, den zutreffenden Satz von Verdampfungsparametern aus dem Datenspeicher auslesen und der Verdampfungssteuerung des Komponentengemisches zugrundelegen. Die Kennung kann Angaben zur Liquidsorte, Nikotinanteil, Raucherprofil, Chargennummer, Produktionsdatum und/oder Mindesthaltbarkeitsdatum enthalten.

Insbesondere bei Anwendung in einem elektronischen Zigarettenprodukt enthält das Komponentengemisch vorteilhaft Nikotin als Wirkstoff, vorzugsweise mit einem Gewichts- oder Massenanteil von 0,1% bis 2%, bevorzugt von 1% bis 2%, besonders bevorzugt 2%, bezogen auf das Gewicht des Komponentengemischs. Im Falle des Wirkstoffs Nikotin ist die zuvor erwähnte höher siedende Komponente beispielsweise Glycerin.

Vorzugsweise enthält das Komponentengemisch Glycerin und/oder 1,2-Propylenglykol als Aerosolbildner. Der Gewichtsanteil von Glycerin und/oder 1,2-Propylenglykol liegt vorzugsweise im Bereich von 50% bis 98%. Der Gewichtsanteil von Glycerin liegt in manchen Ausführungsformen im Bereich zwischen 18% bis 98%, bevorzugt zwischen 26% bis 87%, besonders bevorzugt zwischen 26% bis 50%, und/oder beträgt vorzugsweise mindestens 44 Gewichts-%, weiter vorzugsweise mindestens 50 Gewichts-%, noch weiter vorzugsweise mindestens 55 Gewichts-%, besonders bevorzugt mindestens 60 Gewichts-%, jeweils bezogen auf das Gewicht des Komponentengemischs. Der Gewichtsanteil von 1,2-Propylenglykol liegt vorzugsweise im Bereich zwischen 0% bis 98%, bevorzugt zwischen 20% bis 80%, besonders bevorzugt zwischen 40% bis 70%, bezogen auf das Gewicht des Komponentengemischs.

Das Komponentengemisch kann vorteilhaft Wasser enthalten, vorzugsweise mit einem Gewichtsanteil von 0% bis 30%, bevorzugt von 2% bis 20%, besonders bevorzugt von 4% bis 13%, bezogen auf das Komponentengemisch.

Vorzugsweise enthält der Datenspeicher zu einem ggf. schrittweisen Verdampfen der Komponenten jeweils einzustellende Temperaturen, einzustellende Drücke und/oder einzustellende Zeitintervalle einzelner, ggf. einander folgender Verdampfungsschritte. Der mindestens eine Satz vorgegebener Verdampfungsparameter weist vorteilhaft Daten zu einem über die Zeit schrittweise gestuft abschnittsweise ansteigenden oder fallenden Temperaturprofil, einem schrittweise gestuft abschnittsweise ansteigenden oder fallenden Zeitintervallprofil und/oder zu einem schrittweise gestuft abschnittsweise ansteigenden oder fallenden Druckprofil.

Erfindungsgemäß ist das Verdampfungsintervall in mindestens zwei Phasen unterteilt, wobei in einer ersten Phase eine andere Menge und/oder andere Verdampfungsenergie dem Verdampfer zugesteuert wird als in einer zweiten Phase. Vorzugsweise erstreckt sich dabei die erste Phase über 1/50 bis 2/3, bevorzugt 1/10 bis 1/2 der Dauer des Verdampfungsintervalls. Vorzugsweise ist die Temperatur für den Verdampfer innerhalb einer oder beider Phasen variabel vorgegeben, insbesondere über die Dauer einer oder innerhalb jeder der beiden Phasen zeitweise steigend und/oder zeitweise fallend und/oder zeitweise gleichbleibend.

Insbesondere in der Anwendung als elektronisches Zigarettenprodukt, kurz E-Zigarette, ist der erfindungsgemäße Inhalator vollständig handhaltbar benutzbar, insbesondere in der Art einer Zigarette einhändig handhaltbar benutzbar, sowie tragbar und autark, insbesondere netzunabhängig.

Die Erfindung wird im Folgenden anhand bevorzugter Ausführungsformen unter Bezugnahme auf die beigefügten Figuren erläutert. Dabei zeigt
- Fig. 1: eine Querschnittsansicht eines elektronischen Zigarettenprodukts in einer Ausführungsform der Erfindung;
- Fig. 2: eine Querschnittsansicht einer Kartusche für ein elektronisches Zigarettenprodukt;
- Fig. 3: eine Querschnittsansicht einer Zugabevorrichtung für ein elektronisches Zigarettenprodukt in einer beispielhaften Ausführungsform;
- Fig. 4A-4D: Diagramme zum Verdampfungsverhalten eines Referenz-Komponentengemisches;
- Fig. 5: Diagramme zum Verdampfungsverhalten eines Komponentengemischs mit unterschiedlichen Komponentenanteilen und/oder zu unterschiedlichen Zeitpunkten der Kartuschenentleerung; und
- Fig. 6A-6C: schematische Darstellungen unterschiedlicher Ausführungsformen betreffend den Transport von Flüssigkeit von dem Tank zu der Zugabevorrichtung.

Das elektronische Zigarettenprodukt 10 umfasst ein im Wesentlichen stabförmiges oder zylindrisches Gehäuse 11. In dem Gehäuse 11 ist ein Luftkanal 30 zwischen mindestens einer Lufteinlassöffnung 31 und dem Mundende 32 des Zigarettenprodukts 10 vorgesehen. Das Mundende 32 des Zigarettenprodukts 10 bezeichnet dabei das Ende, an dem der Konsument zwecks Inhalation zieht und dadurch das Zigarettenprodukt 10 mit einem Unterdruck beaufschlagt und eine Luftströmung 34 in dem Luftkanal 30 erzeugt. Mindestens eine Lufteinlassöffnung 31 kann an der Mantelseite des Gehäuses 11 angeordnet sein. Zusätzlich oder alternativ kann mindestens eine Lufteinlassöffnung 31A am entfernten Ende 33 des Zigarettenprodukts 10 angeordnet sein. Das entfernte Ende 33 des Zigarettenprodukts 10 bezeichnet das dem Mundende 32 entgegengesetzte Ende des Zigarettenprodukts 10. Die durch die Einlassöffnung 31 angesaugte Luft wird in dem Luftkanal 30, ggf. über die Schnittstelle bzw. Trennfläche 57 zu der Zugabevorrichtung 20 geleitet. Die Zugabevorrichtung 20 gibt Flüssigkeit 50 aus dem Flüssigkeitstank 18 als Zugabe 40 in Form kleiner Flüssigkeitstropfen als Nebel/Aerosol und/oder gasförmig als Dampf in den Luftstrom 34 zu.

Das Zigarettenprodukt 10 ist vorzugsweise so gestaltet, dass der Zugwiderstand an dem Mundende 32 vorzugsweise im Bereich zwischen 50 mm und 130 mm Wassersäule, weiter vorzugsweise zwischen 80 mm und 120 mm Wassersäule, noch weiter vorzugsweise zwischen 90 mm und 110 mm Wassersäule und optimalerweise zwischen 95 mm und 105 mm Wassersäule liegt. Der Zugwiderstand bezieht sich dabei auf den Druck, der benötigt wird, um Luft durch die volle Länge des Zigarettenprodukts 10 mit einer Rate von 17.5 ml/s bei 22 °C und 101 kPa (760 Torr) zu ziehen, und der in Übereinstimmung mit ISO 6565:2011 gemessen wird.

Das Zigarettenprodukt 10 umfasst einen ersten (Axial-)Abschnitt 13, vorteilhaft am entfernten Ende 33 des Zigarettenprodukts 10, in dem eine elektronische Energieversorgungseinheit 12 mit einem elektrischen Energiespeicher 14 und einer elektrischen/elektronischen Einheit 15 angeordnet ist. Der Energiespeicher 14 erstreckt sich vorteilhaft in axialer Richtung des Zigarettenprodukts 10. Die elektrische/elektronische Einheit 15 ist vorteilhaft seitlich neben dem Energiespeicher 14 angeordnet. Der Energiespeicher 14 kann insbesondere eine elektrochemische Einweg-Batterie oder ein wiederaufladbarer elektrochemischer Akku, z.B. ein Li-Ionen-Akku, sein.

Das Zigarettenprodukt 10 umfasst des Weiteren einen zweiten (Axial-)Abschnitt 16, vorteilhaft am Mundende 32 des Zigarettenprodukts 10, in dem eine Verbrauchseinheit 17 mit einem Flüssigkeitstank 18, einer elektrischen Einheit 19 und einer Zugabevorrichtung 20 angeordnet bzw. anordenbar ist. Der Flüssigkeitstank 18 erstreckt sich vorteilhaft in axialer Richtung des Zigarettenprodukts 10.

Anstelle der getrennten elektrischen/elektronischen Einheiten 15, 19 kann auch eine einheitliche elektrische/elektronische Einheit vorgesehen sein, die entweder in der Energieversorgungseinheit 12 oder in der Verbrauchseinheit 17 angeordnet sein kann. Die Gesamtheit der elektrischen/elektronischen Einheiten des Zigarettenprodukts 10 wird im Folgenden als Steueranordnung 29 bezeichnet.

In dem Gehäuse 11 ist vorteilhaft ein Sensor, beispielsweise ein Drucksensor oder ein Druck- oder Strömungsschalter, angeordnet, wobei die Steueranordnung auf der Grundlage eines von dem Sensor ausgegebenen Sensorsignals einen Betriebszustand des Zigarettenprodukts 10, in dem ein Konsument am Mundende 32 des Zigarettenprodukts 10 zieht, um zu inhalieren, feststellen kann. In diesem Betriebszustand steuert die Steueranordnung 29 die Zugabevorrichtung 20 an, um Flüssigkeit 50 aus dem Flüssigkeitstank 18 als Zugabe 40 in Form kleiner Flüssigkeitstropfen als Nebel/Aerosol und/oder gasförmig als Dampf in den Luftstrom 34 zuzugeben.

Zusätzlich oder alternativ zu dem Strömungsschalter kann das Zigarettenprodukt beispielsweise mittels eines mechanischen Schalters, eines kapazitiven Schalters, der auf Berührung des Gehäuses 11 oder des Mundendes 32 durch den Konsumenten empfindlich ist, oder eines Touchscreens vom Konsumenten ein- und ausschaltbar sein.

Die in dem Flüssigkeitstank 18 gespeicherte, zu dosierende Flüssigkeit (d.h. das flüssige Komponentengemisch) ist beispielsweise eine Mischung aus 1,2-Propylenglykol, Glycerin und/oder Wasser, der ein oder mehrere Aromen (Flavour) und/oder Wirkstoffe, wie beispielsweise Nikotin, zugemischt sein können.

Der den Flüssigkeitstank 18 enthaltende Abschnitt 16 oder die Verbrauchseinheit 17 ist vorteilhaft als vom Konsumenten auswechselbare Kartusche 21, d.h. als Einwegteil ausgeführt. Der Rest des Zigarettenprodukts 10, insbesondere der den Energiespeicher 14 enthaltende Abschnitt 13 ist vorteilhaft als vom Konsumenten wiederverwendbares Grundteil 56, d.h. als Mehrwegteil ausgeführt. Die Kartusche 21 ist vom Konsumenten mit dem Grundteil 56 verbindbar und vom Grundteil 56 lösbar ausgebildet. Zwischen der Kartusche 21 und dem wiederverwendbaren Grundteil 56 ist somit eine Trennfläche bzw. Schnittstelle 57 gebildet. Das Kartuschengehäuse 58 kann einen Teil des Gehäuses 11 des Zigarettenprodukts 10 bilden.

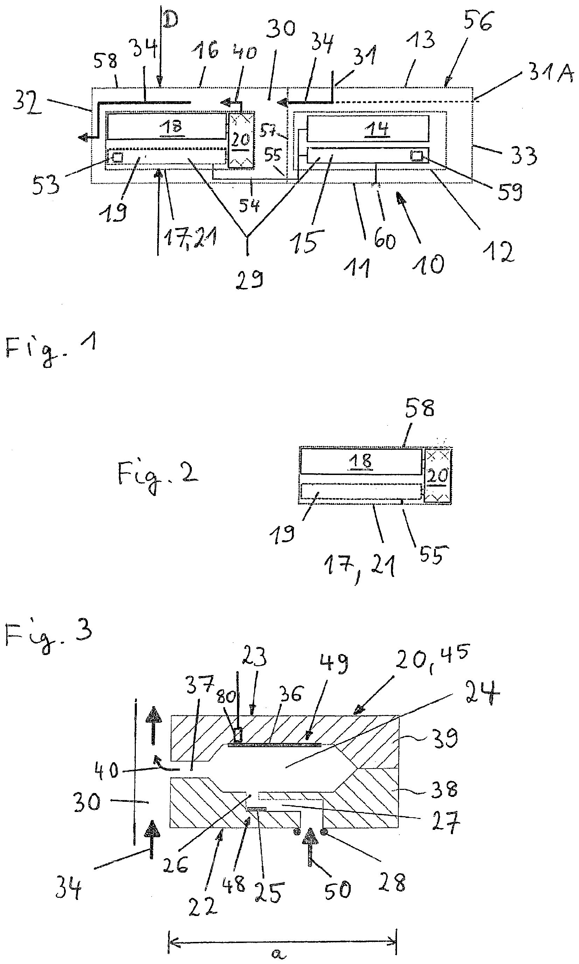

In anderen Ausführungsformen, siehe Fig. 2, ist die Verbrauchseinheit 17 als Kartusche 21 ausgeführt, die in den wiederverwendbaren Grundteil 56 des Zigarettenprodukts 10 durch den Konsumenten einsetzbar und aus diesem entnehmbar ist.

Das Kartuschengehäuse 58 ist in diesem Fall ein von dem Gehäuse 11 des Zigarettenprodukts 10 separates Gehäuse.

Die Kartusche 21 umfasst mindestens den Flüssigkeitstank 18. Die Kartusche 21 kann, wie in Fig. 2 gezeigt, die elektrische/elektronische Einheit 19 umfassen. In anderen Ausführungsformen ist die elektrische/elektronische Einheit 19 ganz oder teilweise fester Bestandteil des Grundteils 56. Ebenso kann die Zugabevorrichtung 20 Teil der Kartusche 21 oder in dem Grundteil 56 angeordnet sein. Die Kartusche 21 kann daher in manchen Ausführungsformen im Wesentlichen nur aus dem Flüssigkeitstank 18 bestehen und ggf. dem Kartuschengehäuse 58, wobei das Kartuschengehäuse 58 alternativ von dem Gehäuse des Flüssigkeitstanks 18 gebildet sein kann, so dass ein separates Kartuschengehäuse 58 entbehrlich sein kann. Vorzugsweise ist eine Füllstandsüberwachung und/oder -anzeige vorgesehen, die es dem Konsumenten ermöglicht, den Füllstand des Flüssigkeitstanks 18 festzustellen.

Die Kartusche 21 kann neben der Verwendung in stabförmigen Zigarettenprodukten 10 auch in anderen Produkten eingesetzt werden, beispielsweise in einer elektronischen Pfeife. Der Energiespeicher 14 ist in der Regel nicht Teil der Kartusche 21, sondern Teil des wiederverwendbaren Grundteils 56.

In einer vorteilhaften Ausführungsform ist der Flüssigkeitstank 18 ein flexibler Beutel. Hierdurch wird mit einfachen Mitteln erreicht, dass der Flüssigkeitstank 18 lageunabhängig und leckagefrei vollständig entleerbar ist. Ein typisches Tankvolumen des Flüssigkeitstanks 18 liegt im Bereich zwischen 0,5 ml und 2 ml. Das Zigarettenprodukt 10 kann vorteilhaft eine Füllstandskontrolle für den Flüssigkeitstank 18 umfassen, die beispielsweise an die Zugzahl gekoppelt sein kann. Der Flüssigkeitstank 18 ist vorzugsweise aus einem inerten und/oder lebensmittelverträglichen bzw. pharmatauglichen Material, insbesondere einem Kunststoff, gefertigt, wobei das Material optisch transparent oder undurchsichtig sein kann.

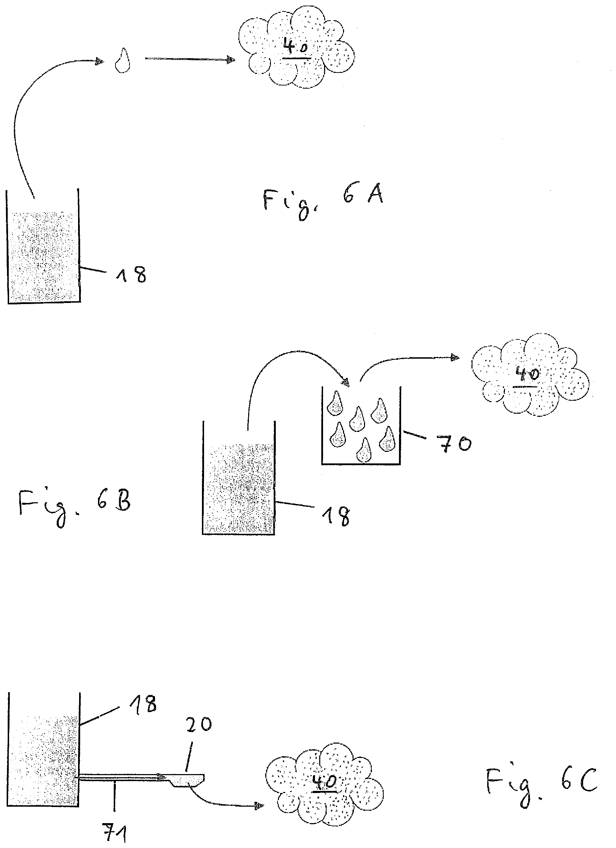

Der Flüssigkeitstank 18 kann an die Zugabevorrichtung 20 mechanisch gekoppelt oder von dieser entkoppelt sein. Im Falle einer mechanischen Kopplung dient die Zugabevorrichtung 20 vorteilhaft als Deckel oder Auslaufschutz für den Flüssigkeitstank 18. Im Falle einer Entkopplung ist insbesondere eine Flüssigkeitsleitung bzw. eine kapillare Verbindung zwischen dem Flüssigkeitstank 18 und der Zugabevorrichtung 20 vorgesehen. Sofern der Flüssigkeitstank 18 von der Zugabevorrichtung 20 trennbar ausgeführt ist, muss dies leckagefrei möglich sein, d.h. der Flüssigkeitstank 18 weist einen Verschließmechanismus auf, der infolge der Trennung des Flüssigkeitstanks 18 von der Zugabevorrichtung 20 automatisch eine Abgabeöffnung des Flüssigkeitstanks 18 flüssigkeitsdicht verschließt, etwa mittels einer federbelasteten Kugel, einem Rückschlagventil oder dergleichen. Unterschiedliche Ausführungsformen betreffend den Transport von Flüssigkeit von dem Flüssigkeitstank 18 zu der Zugabevorrichtung 20 werden später anhand der Figuren 6A bis 6C erläutert.

Das Verhältnis der größten Erstreckung a der mikrosystemtechnischen Einheit 45 (siehe Fig. 3) zum mittleren Durchmesser D des im Wesentlichen stabförmigen Gehäuses 11 im Bereich der Zugabevorrichtung 20 (siehe Fig. 1) ist vorteilhaft kleiner als 0,5.

Eine vorteilhafte Ausführungsform einer erfindungsgemäßen Zugabevorrichtung 20 ist in Fig. 3 gezeigt. Die Zugabevorrichtung 20 umfasst ein Zerstäuberbauteil 22 mit einem Zerstäuber 48 und ein Verdampferbauteil 23 mit einem Verdampfer 49, die in Bezug zu einer Kammer 24 im Inneren der Zugabevorrichtung 20 angeordnet sind.

Der Zerstäuber 48 ist vorzugsweise ein Freistrahl-Zerstäuber nach dem Inkjet- oder Bubblejet-Prinzip, mit einem in einem Flüssigkeitskanal 27 angeordneten Aktuator 25 und einer nachfolgend angeordneten Düse 26, die in die Kammer 24 mündet. Der mit einer geeigneten Ansteuerfrequenz typischerweise im kHz-Bereich und beispielsweise zwischen 10 Hz und 50 kHz, bevorzugt zwischen 100 Hz und 30 kHz, besonders bevorzugt zwischen 1 kHz und 25 kHz elektrisch angesteuerte Aktuator 25 kann ein piezoelektrisches Element oder ein Heizelement sein. Bei Feststellung eines durch Ziehen des Konsumenten verursachten Luftstroms 34 durch den Luftkanal 30 steuert die Steueranordnung 29 den Aktuator 25 an, wobei durch plötzliche Erhitzung (im Falle eines Heizelements) oder durch Erschütterung (im Falle eines Piezo-Elements) die in dem Flüssigkeitskanal 27 befindliche Flüssigkeit in Form von kleinen Tröpfchen aus der Düse 26 in die Kammer 24 geschleudert wird. Der Zerstäuber 48 dient zugleich zur Förderung der Flüssigkeit 50 aus dem Flüssigkeitstank 18 durch den Flüssigkeitskanal 27 sowie der Dosierung der Flüssigkeit in die Kammer 24. Der Zerstäuber 48 kann daher auch als Freistrahl-Dosierer bezeichnet werden.

Der Zerstäuber/Dosierer 48 ist so eingestellt, dass eine vorteilhafte Flüssigkeitsmenge im Bereich zwischen 1 µl bis 10 µl, typischerweise 4 µl pro Zug des Konsumenten, zudosiert wird. Vorzugsweise kann der Zerstäuber/Dosierer 48 hinsichtlich der Flüssigkeitsmenge pro Zug einstellbar sein.

Der Verdampfer 49 weist eine Heizeinrichtung 36 auf, die bei Feststellung eines durch Ziehen des Konsumenten versursachten Luftstroms 34 durch den Luftkanal 30 von der Steueranordnung 29 angesteuert wird, um mittels Strom aus der Energiequelle beheizt zu werden und die aus der Düse 26 austretenden Tröpfchen zu verdampfen, d.h. in den gas- bzw. dampfförmigen Zustand zu versetzen. Zur Erzielung einer optimalen Verdampfung ist die Heizeinrichtung 36 vorzugsweise gegenüberliegend der Düse 26 angeordnet. Die elektrische Heizeinrichtung 36 kann insbesondere eine oder mehrere plattenförmige Heizelemente umfassen.

Die Heizeinrichtung 36 wird von der Steueranordnung 29, insbesondere der elektrischen/elektronischen Einheit 19, bezüglich der Verdampfungstemperatur gesteuert oder geregelt; dies wird im Folgenden noch genauer erläutert. Dies kann vorteilhaft durch eine Steuerung oder Regelung der Heizleistung oder der zugeführten Heizenergie geschehen. Eine vorteilhafte Temperaturregelung kann auf der Grundlage des Messsignals eines Temperatursensors 80, beispielsweise einen Platin-Messwiderstand oder einer widerstands-veränderlichen leitenden Beschichtung des Heizelements 36, erfolgen. Alternativ kann die Temperatur der Heizeinrichtung 36 indirekt bestimmt und zur Temperaturregelung verwendet werden, beispielsweise über die Temperaturabhängigkeit des Widerstands der Heizeinrichtung 36. Die Verdampfungsleistung liegt vorzugsweise im Bereich zwischen 1 W bis 20 W, weiter vorzugsweise im Bereich zwischen 2 W und 10 W. Die Verdampfungstemperatur liegt vorzugsweise im Bereich zwischen 100 °C und 400 °C. Die effektive Fläche der Heizeinrichtung 36 beträgt vorzugsweise zwischen 0,01 mm² bis 9 mm², bevorzugt zwischen 0,05 mm² bis 7 mm², besonders bevorzugt zwischen 0,1 mm² bis 5 mm².

Die Liquidverdampfung erfolgt an der Heizeinrichtung 36 ohne oder mit möglichst geringem Zeitverzug bei exakt einstellbarer und geregelter Heiztemperatur ohne die Gefahr lokaler Überhitzungen. Dies kann vorteilhaft durch eine elektronisch gesteuerte oder geregelte Temperaturbegrenzung der Heizeinrichtung 36, beispielsweise auf die Siedetemperatur einer höher als der Wirkstoff siedenden Komponente, hier 290 °C, erreicht werden.

Vorteilhaft ist die Liquidmenge exakt einstellbar und unabhängig von der Verdampferleistung dosierbar. Überwachungseinrichtungen gewährleisten vorteilhaft, dass nur die vorgesehene Liquidmenge verdampft wird. Mögliche Regelstrategien umfassen die Vorgabe eines Liquid-Fördervolumens, oder Volumenstromprofils mit entsprechender Nachführung, über einen Zug. Dabei wird vorteilhaft die dosierte Liquidmenge für jeden Zug immer vollständig verdampft, um eine Aufkonzentration an der Heizeinrichtung 36 zu vermeiden. Die vorgegebene Temperaturobergrenze wird im Liquid und/oder in der Gasphase zu keiner Zeit überschritten, um eine Schadstoffentstehung durch Zersetzungseffekte der Liquidbestandteile zu vermeiden. Vorteilhaft können unterschiedliche Dampfmengen über die Liquidförderung eingestellt und verdampft werden, ohne die oben genannten Anforderungen an den Verdampfungsprozess zu verletzen. Überwachungseinrichtungen gewährleisten vorteilhaft, dass jeweils nur die exakt eingestellte zu dosierende Liquidmenge verdampft wird. Zu diesem Zweck ist vorteilhaft eine Sensorik zur Überwachung der ordnungsgemäßen Funktion des Zigarettenprodukts 10 vorgesehen.

Die Zerstäuber/Verdampfer-Kombination kann vorteilhaft so eingestellt sein, dass überwiegend Flüssigkeitspartikel mit einem Durchmesser im Bereich zwischen 0.5 µm und 5 µm, bevorzugt zwischen 1 µm und 3 µm entstehen. Partikelgrößen von im Bereich zwischen 0,5 und 2 MMAD (mass median aerodynamic diameter, massen-medianer aerodynamischer Durchmesser), vorzugsweise zwischen 0,7 und 1,5 MMAD, beispielsweise um ca. 1 MMAD können optimal sein. MMAD entspricht einer EU-Norm und wird in µm spezifiziert.

Durchmesser von weniger als 1 µm, beispielsweise im Bereich zwischen 0,7 µm und 1 µm, lassen sich typischerweise nicht durch reines Zerstäuben erreichen, sondern erfordert die Verdampfung der Flüssigkeit mit nachfolgender Rückkondensation aus der Gasphase. Jedoch kann ein (Vor-)Zerstäuben unter Umständen die Verdampfung begünstigen. In einer Ausführungsform ist eine möglichst gleichmäßige Wirkstofffreisetzung für jeden Zug zu gewährleisten. Um unterschiedliche Raucherprofile zu ermöglichen, ist vorteilhaft eine Reserve in der Verdampferleistung vorgesehen.

Da die Kammer 24 insbesondere zur Verdampfung der aus der Düse 26 austretenden Tröpfchen dient, kann die Kammer 24 auch als Verdampferkammer bezeichnet werden. Die Kammer 24 ist im Querschnitt vorteilhaft länglich, wie beispielsweise in Fig. 3 gezeigt, wobei die Düse 26 und das Heizelement 36 vorteilhaft an gegenüberliegenden Längsseiten angeordnet sind. Vorzugsweise senkrecht oder seitlich zu der Austrittsrichtung des Flüssigkeitsstrahls aus der Düse 26 ist eine Austrittsbohrung 37 vorgesehen, durch die der von dem Verdampfer 49 erzeugte Dampf aus der Verdampferkammer 24 austritt, wo er von dem vorzugsweise senkrecht zu der Bohrung 37 verlaufenden Luftstrom 34 mit- und aufgenommen wird.

Da der Aktuator 25 des Zerstäubers 22 und das Heizelement 36 des Verdampfers 49 separat elektrisch mit der Steueranordnung 29 verbunden sind und separat voneinander angesteuert werden, ist eine vorteilhafte funktionale Trennung zwischen der Förderung / Dosierung / Zerstäubung einerseits und der Verdampfung andererseits realisiert.

Der Flüssigkeitskanal 27 ist vorzugsweise mittels einer zwischen der Zugabevorrichtung 20 und dem Flüssigkeitstank 18 angeordneten, die Mündung des Flüssigkeitskanals 27 nach außen umgebenden Dichtung 28 abgedichtet.

Der Dampf bzw. das Aerosol 40 wird entweder der Luftströmung 34 zugeführt, indem diese außen an der Austrittsöffnung 42 der Verdampferkammer 24 vorbeiströmt, siehe Figuren 1 und 3. In alternativen Ausführungsformen durchströmt der Luftstrom 34 die Zugabevorrichtung 20 und der Dampf bzw. das Aerosol 40 wird in der Verdampferkammer 24 von der Luftströmung 34 mit- und aufgenommen. Die Zugabevorrichtung 20 kann fern vom Mundende 32 des Zigarettenprodukts 10, insbesondere im Bereich der Schnittstelle 57 zwischen der Kartusche 21 und dem Grundteil 56 angeordnet sein, wie in dem Ausführungsbeispiel gemäß Fig. 1. Die Zugabevorrichtung 20 kann alternativ nahe am Mundende 32 des Zigarettenprodukts 10 angeordnet sein. Auch eine zum Flüssigkeitstank 18 seitliche Anordnung insbesondere im Bereich der elektrischen/elektronischen Einheit 19 ist möglich.

In der Ausführungsform gemäß Fig. 3 sind sowohl das Zerstäuberbauteil 22 als auch das Verdampferbauteil 23 in Mikrosystem-Technik auf einem Substrat 38, beispielsweise aus einem Polymer, Glas, Keramik, Metall, Halbmetall, z.B. Silizium, Siliziumverbindungen oder Metalloxidverbindungen, ausgeführt. Mikrosystemtechnische Einheiten weisen elektrische und/oder mechanische Strukturen mit Abmessungen im Mikrometer- bzw. Sub-Millimeter-Bereich auf, die in einem einheitlichen Bearbeitungsvorgang in ein Substrat eingearbeitet werden. Im Falle eines Zerstäuberbauteils 22 werden insbesondere der Flüssigkeitskanal 27, der elektrische Aktuator 25 und ggf. in dem Zerstäuberbauteil 22 vorgesehene Sensorik in einem einheitlichen Bearbeitungsvorgang der Mikrosystem-Technologie in das Substrat 38 eingearbeitet. Im Falle eines Verdampferbauteils 23 wird insbesondere das Heizelement 36 und ggf. ein Piezoelement zum Vibrieren des Heizelements 36 und in dem Verdampferbauteil 23 vorgesehene Sensorik in einem einheitlichen Bearbeitungsvorgang der Mikrosystem-Technologie in das Substrat 38 eingearbeitet. In der Ausführungsform gemäß Fig. 3 ist daher die gesamte Zugabevorrichtung 20 als einheitliche mikrosystemtechnische Einheit 45 ausgeführt.

In der Ausführungsform gemäß Fig. 3 ist das Heizelement 36 flach und parallel zu der Oberfläche des Substrats 39, also quasi "liegend" angeordnet. Andere Ausführungsformen sind möglich. In dem Flüssigkeitskanal 27 kann eine Vorheizung mit einem elektrischen Vorheizelement und einer Vorwärmkammer angeordnet sein.

In einer nicht gezeigten Ausführungsform ist nur das Zerstäuberbauteil 22 als mikrosystemtechnische Einheit 45 ausgebildet, während das Substrat 39 des Verdampferbauteils 23 aus einem nichtleitenden Material, insbesondere Glas, Keramik oder einem Kunststoff, gefertigt ist. Dieser Aufbau kann kostengünstiger und somit vorteilhaft sein.

Die Verbrauchseinheit 17 bzw. die Kartusche 21 umfasst vorteilhaft einen nichtflüchtigen Informationsspeicher 53 (siehe Fig. 1) zum Speichern von die Verbrauchseinheit 17 bzw. die Kartusche 21 betreffender Information bzw. Parametern, beispielsweise in Ausführung als EEPROM, RFID oder anderer geeigneter Form. Der Informationsspeicher 53 kann Teil der elektrischen/elektronischen Einheit 19 oder separat davon ausgebildet sein. In dem Informationsspeicher 53 gespeichert ist vorteilhaft Information zum Inhaltsstoff, d.h. zur Zusammensetzung der in dem Flüssigkeitstank 18 gespeicherten Flüssigkeit; Information zum Prozessprofil, insbesondere Leistungs-/Temperatursteuerung; Daten zur Zustandsüberwachung bzw. Systemprüfung, beispielsweise Dichtigkeitsprüfung; Daten betreffend Kopierschutz und Fälschungssicherheit, insbesondere umfassend eine ID zur eindeutigen Kennzeichnung der Verbrauchseinheit 17 bzw. Kartusche 21; Seriennummer, Herstelldatum und/oder Ablaufdatum; und/oder Zugzahl (Anzahl der Inhalationszüge durch den Konsumenten) bzw. der Nutzungszeit. Der Datenspeicher 53 ist vorteilhaft über Kontakte und/oder Leitungen mit der Steuereinrichtung 15 des Grundteils 56 verbunden oder verbindbar.

Zwischen der Verbrauchseinheit 17 bzw. der Kartusche 21 und der Energieversorgungseinheit 12 ist vorteilhaft eine elektrische Verbindung 54 über eine entsprechende elektrische Schnittstelle 55, die eine Auswechslung der Kartusche 21 ermöglicht, vorgesehen. Die elektrische Verbindung 54 dient einerseits dem Datenaustausch zwischen der Verbrauchseinheit 17 bzw. der Kartusche 21 und der Energieversorgungseinheit 12, andererseits der Stromversorgung der Verbrauchseinheit 17 bzw. der Kartusche 21 durch den elektrischen Energiespeicher 14.

Die Energieversorgungseinheit 12 bzw. das Grundteil 56 kann vorteilhaft eine Ladeschnittstelle 60 zum Aufladen des Energiespeichers 14 aufweisen. Die Ladeschnittstelle 60 kann beispielsweise eine Aufladung per Induktion oder direkter elektrischer Ankopplung ermöglichen. Anstelle einer Ladeschnittstelle kann der Energiespeicher auch als Tauschakku oder Tauschbatterie ausgebildet sein, wobei ein entladener Energiespeicher 14 von dem Konsumenten aus dem Zigarettenprodukt 10 entnehmbar und ein geladener Energiespeicher 14 wieder einsetzbar ist. Es sind auch Ausführungsformen mit Einweg-Energiespeicher 14, insbesondere Batterie, ohne Ladeschnittstelle 60 denkbar, wobei das Grundteil nach Entladung des Energiespeichers 14 entsorgt wird.

Bei sämtlichen in den Figuren gezeigten Ausführungsformen weist die Verbrauchseinheit 17 bzw. die Kartusche 21 eine elektrische Steuereinheit 19 und weitere elektrische Komponenten, insbesondere Aktuatoren 25 und Heizelemente 36 auf.

Es sind jedoch auch Ausführungsformen möglich, bei denen die elektrische Steuereinheit 19 und/oder die weiteren elektrischen Komponenten vollständig in dem wiederverwendbaren Grundteil 56 angeordnet sind, so dass die Anzahl der elektrischen Komponenten in der Verbrauchseinheit 17 bzw. Kartusche 21 reduziert sind, oder die Verbrauchseinheit 17 bzw. Kartusche 21 höchstens passive elektrische Komponenten (passiver Datenspeicher 53 wie RFID, Transponder oder dergleichen) umfasst, oder frei von elektrischen Komponenten ist. Diese Ausführungsformen haben den Vorteil, dass eine elektrische Kontaktierung der Kartusche 21 über die elektrische Schnittstelle 55 vorteilhaft entfallen kann.

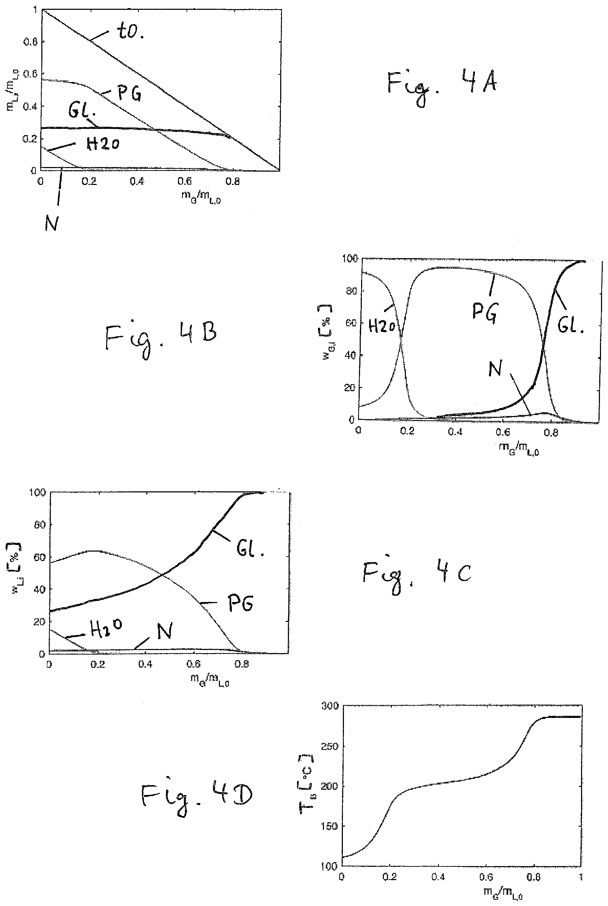

Der erfindungsgemäß aufgefundene, universelle Zusammenhang zwischen Liquidzusammensetzung, Temperaturverhalten während der Verdampfung und Wirkstofffreisetzung wird im Folgenden anhand der Diagramme in den Figuren 4A bis 4D erläutert. Diese Kurven wurden bespielhaft berechnet für ein Referenz-Komponentengemisch mit 63 Gewichts-% Propylenglycol (PG), 29,85 Gewichts-% reinem Glycerin (GI.), 5,15 Gewichts-% Wasser (H₂O) und 2 Gewichts-% Nikotin (N) unter der Annahme einer offenen Verdampfung, d.h. der Dampf (Gasphase) wird kontinuierlich entfernt. Des Weiteren betreffen die Figuren 4A bis 4D den Fall, wo eine anfängliche Flüssigkeitsmasse vollständig verdampft wird. (Dies ist zu unterscheiden von dem als "refill" bezeichneten Fall, wo die Flüssigkeitsmasse durch Nachförderung weiterer Flüssigkeit konstant gehalten wird).

Sämtliche Kurven sind aufgetragen über dem Verhältnis m_{G}/m_{L,0} der verdampften Masse m_{G} bezogen auf die Anfangsmasse m_{L,0} des gesamten Liquids. Figur 4A zeigt die verbleibende Masse m_{L,i} (i = Komponentenindex) in der flüssigen Phase für Propylenglycol (PG), Glycerin (GI.), Wasser (H₂O), Nikotin (N) sowie für die Summe alle Komponenten (to. = total) bezogen auf die Anfangsmasse m_{L,0} des gesamten Liquids. Figur 4B zeigt die Massenanteile w_{G,i} in der Gasphase in Prozent für Propylenglycol (PG), Glycerin (GI.), Wasser (H₂O) und Nikotin (N). Figur 4C zeigt dieselben Massenanteile w_{L,i} in der flüssigen Phase in Prozent. Figur 4D zeigt die Siedetemperatur T_{B} in °C des Komponentengemisches unter der Annahme einer isobaren Verdampfung.

Figur 4B ist besonders instruktiv, da es die Veränderung der Zusammensetzung des vom Konsumenten inhalierten Dampfes (Gasphase) zeigt. Aus Figur 4B ist ersichtlich, dass die Verdampfung der Komponenten Wasser, Propylenglycol und Glycerin im Wesentlichen nicht azeotrop, sondern überwiegend sukzessiv erfolgt, wobei die Reihenfolge durch die jeweiligen Siedetemperaturen bestimmt wird. Die Verdampfung von Nikotin kann an die Verdampfung von Propylenglycol gebunden sein und/oder zwischen der Verdampfung von Propylenglycol und der von Glycerin maximal sein. Jedenfalls ist im zeitlich letzten Abschnitt, wenn im Wesentlichen Glycerin verdampft wird, das Nikotin im Wesentlichen bereits vollständig verdampft, siehe auch Figur 4C. Es ist ersichtlich, dass die Konzentration und Wirksamkeit des Wirkstoffs Nikotin, sowie die Zeitdauer der Wirkstoffbeigabe, durch den Anteil an Glycerin (allgemein der oder einer höher siedenden Komponente) in dem Komponentengemisch gesteuert werden kann.

Wenn die Verdampfertemperatur (Temperatur der Heizeinrichtung 36) vorteilhaft etwa im Bereich zwischen der Siedetemperatur des Wirkstoffs und der Siedetemperatur der oder einer höher siedenden Komponente gewählt wird, hier beispielsweise im Bereich zwischen 250 °C und 290 °C, vorzugsweise im Bereich zwischen 260 °C und 290 °C, weiter im Bereich zwischen 270 °C und 290 °C, beispielsweise bei etwa 280 °C, folgt der Temperaturverlauf der Heizeinrichtung 36 der in Figur 4D gezeigten Kurve bis zum Erreichen der eingestellten Maximaltemperatur (beispielsweise 280 °C), die jedoch nicht mehr ausreicht, um die höher siedende Komponente (hier Glycerin) effektiv zu verdampfen. Der Konsument merkt an der fehlenden Dampfentwicklung, dass der Anteil des Wirkstoffs im Kartuscheninhalt aufgebraucht ist, und wechselt die verbrauchte Kartusche 21, die noch einen erheblichen Anteil der höher siedenden (Dummy-) Komponente (Glycerin) enthalten kann, gegen eine neue Kartusche 21 aus.

Die in den Figuren 4A bis 4D gezeigten universellen Kurven lassen sich für jedes relevante Komponentengemisch berechnen oder per Kalibrierung vermessen. Die optimalen Verdampfungsparameter lassen sich für jedes relevante Komponentengemisch aus den universellen Verdampfungskurven ableiten und werden herstellerseitig in einem Datenspeicher 59 des Grundteils 56 abgelegt. Der Datenspeicher 59 ist vorteilhaft in der elektronischen Einheit 15 des Grundteils 56 vorgesehen. Beim Einsetzen einer neuen Kartusche 21 in das Zigarettenprodukt 10 liest die elektronische Steuereinrichtung 15 des Grundteils 56 die Kennung der Kartusche 21, die in dem Datenspeicher 53 der Kartusche 21 hinterlegt ist und das in der Kartusche 21 enthaltene Komponentengemisch eindeutig beschreibt, aus dem Datenspeicher 53 aus, lädt den der Kennung eindeutig zugeordneten Verdampfungsparametersatz aus dem Datenspeicher 59 des Grundteils 56 aus, und steuert den Zerstäuber 48 und/oder den Verdampfer 49 auf der Grundlage des eingelesenen Verdampfungsparametersatzes. Auf diese Weise wird für jedes Komponentengemisch eine optimale Verdampfung automatisch sichergestellt. Es ist auch möglich, die Verdampfungsparameter in dem Datenspeicher 53 der Kartusche 21 von der elektronischen Steuereinrichtung 15 auslesbar zu hinterlegen. In diesem Fall kann eine Hinterlegung in dem Datenspeicher 59 des Grundteils 56 entbehrlich sein.

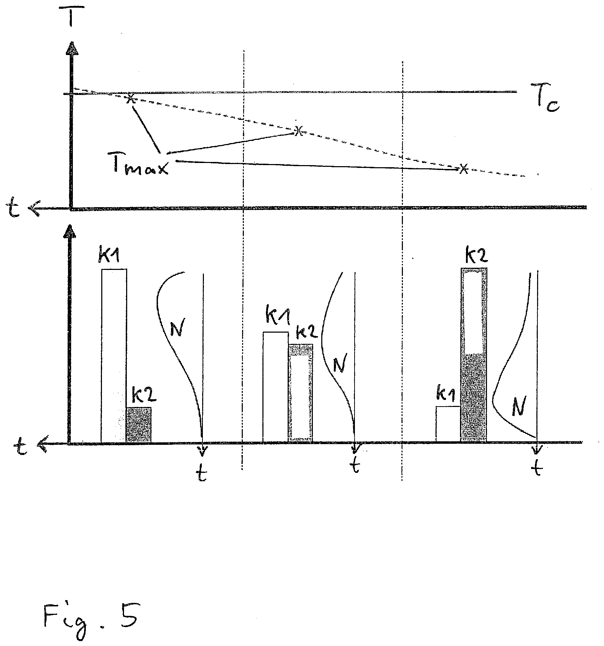

Das Diagramm in Figur 5 verdeutlicht einerseits den gezielten Einsatz der Liquidzusammensetzung zur Beeinflussung der Wirkstofffreisetzung bei gegebener bzw. eingestellter Grenztemperatur (Maximaltemperatur) des Verdampfers. Der untere Teil von Figur 5 zeigt drei unterschiedliche Komponentengemische. Im linken Fall überwiegt die Komponente K1 gegenüber der Komponente K2. Im mittleren Fall haben die Komponenten K1 und K2 etwa vergleichbare Anteile. Im rechten Fall überwiegt die Komponente K2 gegenüber der Komponente K1. Die Komponente K1 könnte beispielsweise höher, die Komponente K2 niedriger als der Wirkstoff sieden. Aus dem oberen Teil von Figur 5 ist ersichtlich, dass sich die Maximaltemperatur des Verdampfers Tₘₐₓ nach den jeweiligen Anteilen des Komponentengemisches ändert. In jedem Fall sollte die Maximaltemperatur des Verdampfers Tₘₐₓ unterhalb der kritischen Temperatur T_{c} liegen, welche die Grenze für Schadstofffreisetzung markiert.

Die Kurvenverläufe im unteren Teil der Figur 5 zeigen den zeitlichen Verlauf der Wirkstofffreisetzung. Im linken Fall wird der Wirkstoff (etwa Nikotin N) überwiegend relativ früh freigesetzt, im mittleren Fall liegt das Maximum der Wirkstofffreisetzung in einem mittleren zeitlichen Bereich, während im rechten Fall der Wirkstoff überwiegend relativ spät freigesetzt wird. Dies verdeutlicht, wie der zeitliche Verlauf der Wirkstofffreisetzung durch Wahl der Anteile der Komponenten des Komponentengemisches gezielt eingestellt bzw. gesteuert werden kann.

Andererseits kann die Figur 5 auch eine zeitliche Änderung der Komponentenanteile während der Kartuschenentleerung infolge partieller Entmischung des Komponentengemisches darstellen. In diesem Fall ist die horizontale Achse in Figur 5 eine Zeitachse, die hier von rechts nach links verläuft, entsprechend einer mit der Zeit ansteigenden Maximaltemperatur Tₘₐₓ.

Wie im Folgenden erläutert wird, beschreibt die Figur 5 im Zusammenhang mit den Figuren 6A bis 6C drei wesentlich unterschiedliche Betriebsarten bzw. Technologien des Inhalators.

Die Figuren 6A bis 6C verdeutlichen, wie durch unterschiedlichen Transport der Flüssigkeit von dem Flüssigkeitstank 18 zu der Zugabevorrichtung 20 bzw. zu dem Verdampfer 49 das Verdampfungsverhalten und die Wirkstofffreisetzung gezielt beeinflusst werden kann.

Figur 6A betrifft eine tröpfchengenaue aktive Förderung der Flüssigkeit von dem Flüssigkeitstank 18 zu dem Verdampfer 49 zur Erzeugung des Dampfs 40. Ein typisches Ausführungsbeispiel hierfür wäre ein Freistrahl-Zerstäuber 48 nach dem Inkjet- oder Bubblejet-Prinzip. Diese Variante führt zu einer definierten, gleichmäßigen Wirkstofffreisetzung bzw. -dosierung je Zug, insbesondere unabhängig von der jeweiligen Flüssigkeitszusammensetzung. Die einmalig oder gepulst zugeführte Liquidmenge wird vollständig verdampft. Durch die vorteilhafte Steuerung oder Regelung der Heizeinrichtung 36 auf die maximal benötigte Temperatur Tₘₐₓ zur Verdampfung der höchstsiedenden Einzelkomponente des Liquids bleibt diese nach dem Verdampfungsvorgang frei von Liquidrückständen. Die Zusammensetzung des Dampfes entspricht bei jedem Zug der Ausgangszusammensetzung des Liquids.

Dem entsprechend wird also in Figur 5 die Wirkstofffreigabe in Abhängigkeit des Volumen-Anteils einer höhersiedenden Komponente für drei unterschiedliche, unabhängige Liquids gezeigt. Wann immer man pro Inhalation/Raucherzug die Flüssigkeitszusammensetzung konstant hält, wird auch die Wirkstofffreigabe bei jedem Zug gleichbleiben. In der technologischen Umsetzung entspricht das der Fig. 6A, in der man immer einen identisch zusammengesetzten Tropfen der Verdampfung zuführt. Im eigentlichen Reservoir findet somit keine Entmischung statt.

Figur 6B betrifft eine aktive Förderung zum Verdampfer 49 über ein Zwischenreservoir 70, das entweder in der Kartusche 21 oder in dem Grundteil 56 angeordnet ist.

Diese Variante führt zu einer kontrollierten Wirkstofffreisetzung über eine definierte Zugzahl bei Verdampfung aus dem Zwischenreservoir 70. Im Zwischenreservoir 70 findet eine Entmischung der Flüssigkeit abhängig von der Flüssigkeitszusammensetzung und der erlaubten Maximaltemperatur Tₘₐₓ des Verdampfers statt. Der Verdampfer wird bei dieser Variante vorteilhaft auf eine Temperatur unterhalb oder gleich der benötigten Temperatur Tₘₐₓ zur Verdampfung der höchstsiedenden Einzelkomponente des Liquids gesteuert oder geregelt. Die Liquidzusammensetzung bestimmt die Konzentration und zeitliche Freisetzung des Wirkstoffs im Dampf. Die dem Zwischenreservoir 70 einmalig oder gepulst zugeführte Liquidmenge wird während der definierten Zugzahl vollständig verdampft.

Figur 6C betrifft eine passive Förderung über eine Transporteinrichtung 71, beispielsweise eine Rohrleitung, zu der Zugabevorrichtung 20 bzw. dem Verdampfer 49. Diese Variante führt zu einer kontrollierten Wirkstofffreisetzung, insbesondere durch Regelung der Verdampfungstemperatur auf einen Wert unterhalb der benötigten Temperatur Tₘₐₓ zur Verdampfung der höchstsiedenden Einzelkomponente des Liquids. Im Flüssigkeitstank 18 findet eine Entmischung der Flüssigkeit abhängig von der Flüssigkeitszusammensetzung und der erlaubten Maximaltemperatur Tₘₐₓ des Verdampfers statt. Hierdurch wird der Wirkstoff gezielt ausgetrieben, ohne dass der gesamte Inhalt des Liquidreservoirs verdampft. Die Wirkstoffkonzentration im Dampf wird gegenüber der Wirkstoffkonzentration im Liquid erhöht. Bevor die höchstsiedende Einzelkomponente in die Gasphase übergeht, ist kein Wirkstoff in dem Flüssigkeitstank 18 mehr vorhanden. Durch die Begrenzung der Verdampfungstemperatur schaltet das System ab, bevor Schadstoffe emittiert werden können.

Sobald also in einem (Zwischen-)Reservoir 70, 18 eine Entmischung stattfinden kann, Fig. 6B bzw. 6C, beschreibt die Fig. 5 in diesem Fall die langsame zeitliche Entmischung des Liquids (in Figur 5 von rechts nach links) an drei beispielhaften Zustandspunkten, also auf der übergeordneten horizontalen Zeitskala die Veränderung des Zeitpunkts der Wirkstofffreigabe pro Zug und parallel dazu den Anstieg der für die Verdampfung benötigten Systemtemperatur, da die Temperatur proportional zum Anstieg des Anteils der höchstsiedenden Komponente verläuft, analog zu Fig. 4C und 4D.

Die in Fig. 4A bis 4D beschriebene Erkenntnis erlaubt damit, bei vorhandener Hardware, Fig. 6A bis 6C, drei verschiedene Regelungskonzepte für die jeweilige entsprechende Hardware umzusetzen.

## Patentansprüche

1. Verfahren zum Betreiben eines Inhalators umfassend ein Gehäuse (11) mit einem Mundende (32), mindestens einer Lufteinlassöffnung (31) und einem in dem Gehäuse (11) zwischen der mindestens einen Lufteinlassöffnung (31) und dem Mundende (32) verlaufenden Luftkanal (30), einen Flüssigkeitsspeicher (19), der ein Komponentengemisch enthält, eine Aufnahme für den Flüssigkeitsspeicher (18), einen elektrischen Energiespeicher (14), eine Zugabevorrichtung (20), mit der Dampf und/oder Aerosol von aus dem Flüssigkeitsspeicher (18) entnommenem Komponentengemisch (50) erzeugt wird, und der Dampf und/oder das Aerosol (40) zu einem in dem Luftkanal (30) strömenden Luftstrom (34) zugegeben wird, wobei die Zugabevorrichtung (20) einen ansteuerbaren Verdampfer (49) aufweist, und mit einer Steuereinrichtung (15; 29) zum Steuern der Zugabevorrichtung (20), wobei die Steuereinrichtung (15; 29) mit einem Datenspeicher (53; 59) verbunden ist, in dem zum Verdampfen des Komponentengemischs in dem Verdampfer (49) mindestens ein Satz vorgegebener Verdampfungsparameter abrufbar hinterlegt wird, wobei der mindestens eine Satz vorgegebener Verdampfungsparameter aus dem Flüssigphase-Gasphase-Gleichgewichts Diagramm des Komponentengemisches abgeleitet wird, wobei eine zu jedem Zeitpunkt der Verdampfung abgerufene Heizleistung zur Steuerung der Verdampfung des Komponentengemisches genutzt wird, **dadurch gekennzeichnet, dass** eine momentane Dampfzusammensetzung gezielt eingestellt wird, indem die Steuerung der Verdampfung des Komponentengemisches mittels des hinterlegten Satzes von Verdampfungsparametern erfolgt.

2. Verfahren nach Anspruch 1, **dadurch gekennzeichnet, dass** anhand der zu jedem Zeitpunkt der Verdampfung abgerufenen Heizleistung auf eine momentane Liquid- bzw. Dampfzusammensetzung rückgeschlossen wird, wobei die so erkannte momentane Liquid- bzw. Dampfzusammensetzung zur Steuerung der Verdampfung des Komponentengemisches genutzt wird.

3. Verfahren nach einem der vorangehenden Ansprüche, wobei der Verdampfer (49) eine mittels Strom beheizbare Heizeinrichtung (36) aufweist, **dadurch gekennzeichnet, dass** der mindestens eine Satz vorgegebener Verdampfungsparameter Daten zu einer oder mehreren aus der Gruppe der folgenden Größen enthält:
- eine einzustellende Temperatur der Heizeinrichtung (36);
- eine einzustellende Dauer einzelner Verdampfungsschritte und/oder des gesamten Verdampfungsvorgangs;
- einen einzustellenden Druck in einem Zerstäuber (48);
- eine bereitzustellende Verdampfungsenergie für einen Verdampfer (49).

4. Verfahren nach einem der vorangehenden Ansprüche, wobei der Verdampfer (49) eine mittels Strom beheizbare Heizeinrichtung (36) aufweist, **dadurch gekennzeichnet, dass** der mindestens eine Satz vorgegebener Verdampfungsparameter Daten zu einer oder mehreren aus der Gruppe der folgenden Größen enthält:
- eine minimale Temperatur und/oder eine maximale Temperatur der Heizeinrichtung (36);
- einen minimalen Druck und/oder einen maximalen Druck in einem Zerstäuber (48);
- eine minimale Dauer und/oder eine maximale Dauer einzelner Verdampfungsschritte und/oder des gesamten Verdampfungsvorgangs.

5. Verfahren nach einem der vorangehenden Ansprüche, **dadurch gekennzeichnet, dass** die Steuereinrichtung (29) die Temperatur des Verdampfers (49) mindestens über ein einem Inhalationszug entsprechendes Verdampfungsintervall oberhalb einer ersten charakteristischen Temperatur, insbesondere der Siedetemperatur einer Komponente des Komponentengemisches, hält.

6. Verfahren nach Anspruch 5, **dadurch gekennzeichnet, dass** der vorgegebene Temperaturwert im Bereich zwischen 1 % bis 90 %, vorzugsweise 3 % bis 70 %, besonders bevorzugt 5 % bis 50 % oberhalb der ersten charakteristischen Temperatur liegt.

7. Verfahren nach einem der vorangehenden Ansprüche, **dadurch gekennzeichnet, dass** die Steuereinrichtung (29) die Temperatur des Verdampfers (49) mindestens über ein Verdampfungsintervall unterhalb einer zweiten charakteristischen Temperatur, insbesondere der Siedetemperatur einer Komponente des Komponentengemisches, hält.

8. Verfahren nach Anspruch 7, **dadurch gekennzeichnet, dass** die zweite charakteristische Temperatur die Siedetemperatur einer höchstsiedenden Komponente des Komponentengemisches ist.

9. Verfahren nach einem der Ansprüche 5 bis 8, **dadurch gekennzeichnet, dass** das Verdampfungsintervall eine Dauer von 1 bis 12 Sekunden, bevorzugt 2 bis 8 Sekunden aufweist.

10. Verfahren nach einem der vorangehenden Ansprüche, **dadurch gekennzeichnet, dass** der Verdampfer (49) eine Heizeinrichtung (36) umfasst, die auf eine vorgegebene Temperatur eingestellt wird.

11. Verfahren nach Anspruch 10, **dadurch gekennzeichnet, dass** der Verdampfer (49) einen Temperatursensor (80) aufweist, mit dem die Temperatur der Heizeinrichtung (36) gemessen wird und/oder die Temperatur der Heizeinrichtung (36) gesteuert oder geregelt wird.

12. Verfahren nach Anspruch 10 oder 11, **dadurch gekennzeichnet, dass** die vorgegebene Temperatur innerhalb eines Verdampfungsintervalls variabel ist, insbesondere über die Dauer eines Verdampfungsintervalls zeitweise steigend und/oder zeitweise fallend und/oder zeitweise gleichbleibend.

13. Verfahren nach einem der vorangehenden Ansprüche, **dadurch gekennzeichnet, dass** eine Maximal-Temperatur des Verdampfers höchstens 350 °C, bevorzugt höchstens 300 °C und besonders bevorzugt höchstens 290 °C beträgt.

14. Verfahren nach einem der vorangehenden Ansprüche, **dadurch gekennzeichnet, dass** dem Flüssigkeitsspeicher (18) eine maschinell auslesbare Kennung des Komponentengemisches zugeordnet ist, die bevorzugt in dem Datenspeicher (53) gespeichert wird.

15. Verfahren nach einem der vorangehenden Ansprüche, **dadurch gekennzeichnet, dass** eine gezielte Beeinflussung des Verdampfungsverhaltens und der Wirkstofffreisetzung mittels geeignet gestaltetem Transport der Flüssigkeit von dem Flüssigkeitstank (18) zu der Zugabevorrichtung (20) stattfindet.

16. Verfahren nach Anspruch 15, **dadurch gekennzeichnet, dass** der Transport der Flüssigkeit von dem Flüssigkeitstank (18) zu der Zugabevorrichtung (20) über ein Zwischenreservoir (70) erfolgt.

17. Verfahren nach Anspruch 15 oder 16, **dadurch gekennzeichnet, dass** der Verdampfer (49) mit dem Flüssigkeitsspeicher (18) über eine Leitung (71) permanent oder zum Bilden vorgegebener Flüssigkeitsportionen unterbrechbar fluidleitend verbunden ist.

18. Verfahren nach einem der Ansprüche 15 bis 17, **dadurch gekennzeichnet, dass** die Zugabevorrichtung (20) einen Tropfenzerstäuber (48) aufweist, über den die Flüssigkeit tropfenweise auf die beabstandet angeordnete Heizeinrichtung (36) geleitet wird.

## Claims

1. Method for operating an inhaler comprising a housing (11) with a mouth end (32), at least one air inlet opening (31) and an air channel (30) extending in the housing between the at least one air inlet opening (31) and the mouth end (32), a fluid reservoir (19) storing a component mixture, a receiving area for the fluid reservoir (18), an electrical energy storage (14), a supply device (20) which generates vapor and/or aerosol from the component mixture (50) extracted from the fluid reservoir (18), and adds the vapor and/or the aerosol (40) to an air flow (34) flowing in the air channel (30), wherein the supply device (20) comprises a controllable evaporator (49), and a control device (15; 29) for controlling the supply device (20), wherein the control device (15; 29) is connected to a data store (53; 59) in which at least one set of predefined evaporating parameters is stored and can be called upon for evaporating the component mixture in the evaporator (49), wherein the at least one set of predefined evaporating parameters is deduced from the liquid phase-gas phase equilibrium diagram of the component mixture, wherein a heating power retrieved at any time during the evaporation is used to control the evaporation of the component mixture, **characterized in that** an instantaneous vapor composition is specifically adjusted by controlling the evaporation of the component mixture by means of the stored set of evaporation parameters.

2. Method according to claim 1, **characterized in that** the heating power retrieved at any time during the evaporation is used to deduce an instantaneous liquid or vapor composition, wherein the instantaneous liquid or vapor composition detected in this way is used to control the evaporation of the component mixture.

3. Method according to any of the preceding claims, wherein the evaporator (49) comprises a heating device (36) which can be heated by means of electrical current, **characterized in that** the at least one set of predefined evaporating parameters contains data regarding one or more variables from the following group of variables:
- a temperature of the heating device (36) to be adjusted;
- a duration of individual evaporation steps and/or of the entire evaporation process, which is to be adjusted;
- a pressure to be adjusted in an atomizer (48);
- an evaporation energy to be provided for an evaporator (49) .

4. Method according to any one of the preceding claims, wherein the evaporator (49) comprises a heating device (36) which can be heated by means of electrical current, **characterized in that** the at least one set of predefined evaporating parameters contains data regarding one or more variables from the following group of variables:
- a minimum temperature and/or a maximum temperature of the heating device (36);
- a minimum pressure and/or a maximum pressure in an atomizer (48);
- a minimum duration and/or a maximum duration of individual evaporation steps and/or of the entire evaporation process.

5. Method according to any one of the preceding claims, **characterized in that** the control device (29) keeps the temperature of the evaporator (49), at least over an evaporation interval corresponding to an inhalation puff, above a first characteristic temperature, in particular the boiling temperature of a component of the component mixture.

6. Method according to Claim 5, **characterized in that** the predefined temperature value lies in the range between 1% and 90%, preferably 3% to 70%, particularly preferably 5% to 50% above the first characteristic temperature.

7. Method according to any one of the preceding claims, **characterized in that** the control device (29) keeps the temperature of the evaporator (49), at least over an evaporation interval, below a second characteristic temperature, in particular the boiling temperature of a component of the component mixture.

8. Method according to Claim 7, **characterized in that** the second characteristic temperature is the boiling temperature of a highest-boiling component of the component mixture.

9. Method according to any one of Claims 5 to 8, **characterized in that**
the evaporation interval has a duration of 1 to 12 seconds, preferably 2 to 8 seconds.

10. Method according to any one of the preceding claims, **characterized in that** the evaporator (49) comprises a heating device (36) which is adjusted to a predefined temperature.

11. Method according to Claim 10, **characterized in that** the evaporator (49) comprises a temperature sensor (80) which measures the temperature of the heating device (36) and/or controls or regulates the temperature of the heating device (36).

12. Method according to Claim 10 or 11, **characterized in that** the predefined temperature is variable within an evaporation interval, in particular it intermittently increases and/or intermittently falls and/or is intermittently constant over the duration of an evaporation interval.

13. Method according to any one of the preceding claims, **characterized in that** a maximum temperature of the evaporator is at most 350°C, preferably at most 300°C and, particularly preferably, at most 290°C.

14. Method according to any one of the preceding claims, **characterized in that** a machine-readable identification of the component mixture is assigned to the fluid reservoir (18), which is preferably stored in the data store (53).

15. Method according to any one of the preceding claims, **characterized in that** the evaporation behavior is influenced in a deliberate manner, and the active ingredient released, by means of a suitably designed transport of the fluid from the fluid reservoir (18) to the supply device (20).

16. Method according to Claim 15, **characterized in that** the fluid is transported from the fluid reservoir (18) to the supply device (20) by means of an intermediate reservoir (70).

17. Method according to Claim 15 or 16, **characterized in that** the evaporator (49) is connected or can be connected to the fluid reservoir (18) permanently by means of a line (71), or interruptibly in a fluid-conducting manner in order to form predefined fluid portions.

18. Method according to any one of Claims 15 to 17, **characterized in that** the supply device (20) comprises a drop atomizer (48) via which the fluid is conducted drop by drop to the heating device (36) which is arranged at a distance.

## Revendications

1. Procédé pour manier un inhalateur comprenant un boîtier (11) avec une extrémité buccale (32), au moins une ouverture d'entrée d'air (31) et un conduit d'air (30) s'étendant dans le boîtier (11) entre ladite au moins une ouverture d'entrée d'air (31) et l'extrémité buccale (32), un réservoir de liquide (19) qui contient un mélange de composants, un logement pour le réservoir de liquide (18), un accumulateur d'énergie électrique (14), un dispositif d'addition (20), avec lequel de la vapeur et/ou un aérosol de mélange de composants (50) prélevé dans le réservoir de liquide (18) est produit, et la vapeur et/ou l'aérosol (40) sont additionnés à un courant d'air (34) circulant dans le conduit d'air (30), le dispositif d'addition (20) présentant un vaporisateur (49) pouvant être commandé, et avec un moyen de commande (15 ; 29) pour commander le dispositif d'addition (20), le moyen de commande (15 ; 29) étant relié à une mémoire de données (53 ; 59) dans laquelle, pour la vaporisation du mélange de composants dans le vaporisateur (49), au moins un jeu de paramètres de vaporisation prédéfinis est enregistré de manière à pouvoir être appelé, ledit au moins un jeu de paramètres de vaporisation prédéfinis étant dérivé du diagramme d'équilibre phase liquide-phase gazeuse du mélange de composants, une puissance de chauffage appelée à chaque instant de la vaporisation étant utilisée pour commander la vaporisation du mélange de composants,
**caractérisé en ce qu'**une composition de vapeur instantanée est réglée de manière ciblée par le fait que la commande de la vaporisation du mélange de composants s'effectue au moyen du jeu de paramètres de vaporisation enregistré.

2. Procédé selon la revendication 1, **caractérisé en ce que** de la puissance de chauffage appelée à chaque instant de la vaporisation est déduite une composition instantanée de liquide ou de vapeur, la composition instantanée de liquide ou de vapeur ainsi obtenue étant utilisée pour commander la vaporisation du mélange de composants.

3. Procédé selon l'une des revendications précédentes, dans lequel le vaporisateur (49) présente un moyen de chauffage (36) pouvant être chauffé par un courant, **caractérisé en ce que** ledit au moins un jeu de paramètres de vaporisation prédéfinis contient des données relatives à une ou plusieurs grandeurs choisies dans le groupe constitué par :
- une température à régler du moyen de chauffage (36) ;
- une durée à régler de différentes étapes de vaporisation et/ou du processus de vaporisation complet ;
- une pression à régler dans un atomiseur (48) ;
- une énergie de vaporisation à fournir à un vaporisateur (49).

4. Procédé selon l'une des revendications précédentes, dans lequel le vaporisateur (49) présent un moyen de chauffage (36) pouvant être chauffé par un courant, **caractérisé en ce que** ledit au moins un jeu de paramètres de vaporisation prédéfinis contient des données relatives à une ou plusieurs grandeurs choisies dans le groupe constitué par :
- une température minimale et/ou une température maximale du moyen de chauffage (36) ;
- une pression minimale et/ou une pression maximale dans un atomiseur (48) ;
- une durée minimale et/ou une durée maximale des différentes étapes de vaporisation et/ou du processus de vaporisation complet.

5. Procédé selon l'une des revendications précédentes, **caractérisé en ce que** le moyen de commande (29) maintient la température du vaporisateur (49) au-dessus d'une première température caractéristique, en particulier la température d'ébullition d'un composant du mélange de composants, pendant au moins un intervalle de vaporisation correspondant à une inhalation.

6. Procédé selon la revendication 5, **caractérisé en ce que** la valeur de température prédéfinie se situe dans la plage comprise entre 1 % à 90 %, de préférence entre 3 % à 70 %, de manière particulièrement préférée entre 5 % à 50 % au-dessus de la première température caractéristique.

7. Procédé selon l'une des revendications précédentes, **caractérisé en ce que** le moyen de commande (29) maintient la température du vaporisateur (49) en dessous d'une deuxième température caractéristique, en particulier la température d'ébullition d'un composant du mélange de composants, pendant au moins un intervalle de vaporisation.

8. Procédé selon la revendication 7, **caractérisé en ce que** la deuxième température caractéristique est la température d'ébullition d'un composant au point d'ébullition le plus élevé du mélange de composants.

9. Procédé selon l'une des revendications 5 à 8, **caractérisé en ce que** l'intervalle de vaporisation présente une durée de 1 à 12 secondes, de préférence de 2 à 8 secondes.

10. Procédé selon l'une des revendications précédentes, **caractérisé en ce que** le vaporisateur (49) comprend un moyen de chauffage (36) qui est réglé à une température prédéfinie.

11. Procédé selon la revendication 10, **caractérisé en ce que** le vaporisateur (49) présente un capteur de température (80) permettant de mesurer la température du moyen de chauffage (36) et/ou de commander ou réguler la température du moyen de chauffage (36).

12. Procédé selon la revendication 10 ou 11, **caractérisé en ce que** la température prédéfinie est variable à l'intérieur d'un intervalle de vaporisation, en particulier augmente temporairement et/ou diminue temporairement et/ou reste temporairement constante pendant la durée d'un intervalle de vaporisation.

13. Procédé selon l'une des revendications précédentes, **caractérisé en ce qu'**une température maximale du vaporisateur est d'au plus 350 °C, de préférence d'au plus 300 °C et de manière particulièrement préférée d'au plus 290 °C.

14. Procédé selon l'une des revendications précédentes, **caractérisé en ce qu'**un identifiant du mélange de composants, lisible par machine, est associé au réservoir de liquide (18) et est de préférence enregistré dans la mémoire de données (53).

15. Procédé selon l'une des revendications précédentes, **caractérisé en ce qu'**une influence ciblée est exercée sur le comportement de vaporisation et la libération des substances actives au moyen d'un transport du liquide conçu de manière appropriée du réservoir de liquide (18) vers le dispositif d'addition (20).

16. Procédé selon la revendication 15, **caractérisé en ce que** le transport du liquide du réservoir de liquide (18) vers le dispositif d'addition (20) s'effectue via un réservoir intermédiaire (70).

17. Procédé selon la revendication 15 ou 16, **caractérisé en ce que** le vaporisateur (49) est relié fluidiquement au réservoir de liquide (18) par une conduite (71) de manière permanente ou interruptible pour former des portions de liquide prédéfinies.

18. Procédé selon l'une des revendications 15 à 17, **caractérisé en ce que** le dispositif d'addition (20) présente un atomiseur goutte à goutte (48) par lequel le liquide est conduit goutte à goutte vers le moyen de chauffage (36) disposé à distance.
